# EUROPEAN PATENT SPECIFICATION

(11) **EP 1 409 045 B1**
(45) Date of publication and mention of the grant of the patent: **01.04.2009**
(21) Application number: 02751322.5
(22) Date of filing: 25.07.2002
(51) Int. Cl.: A61M 5/30

(54) **PARTICLE CASSETTE, METHOD AND KIT THEREFOR**
TEILCHENKASSETTE, VERFAHREN UND KIT DAFÜR
CASSETTE DE PARTICULES, PROCEDE ET DISPOSITIF CORRESPONDANT

(30) Priority: 26.07.2001 US 307986 P; 26.07.2001 US 916176
(43) Date of publication of application: 21.04.2004
(73) Proprietor: PowderJect Research Limited, Oxford OX4 4GA (GB)
(72) Inventor: SMITH, Edward, Robert, Oxford OX4 4GA (GB); KENDALL, Mark, Anthony, Fernance, Oxford OX4 4GA (GB); WATSON, John, Oxford OX4 4GA (GB); FORD, Graham, Oxford OX4 4GA (GB)
(74) Representative: Hadjadj, Laurent
(86) International application number: PCT/GB2002/003395
(87) International publication number: WO 2003/011379

(56) References cited:
- EP-A- 0 951 917
- US-A- 5 947 928

## Description

The present invention relates generally to the retention of particles prior to the needleless injection of those particles in a gas stream. More specifically, the present invention relates to particle cassettes having a pair of membranes which retain the particles in a chamber therebetween.

Needleless syringe devices are known from WO 94/24263. In this document, a needleless syringe is disclosed which entrains particles in a gas stream accelerated through a nozzle so that the particles may be injected into a target, such as human skin or other cells. For many applications, there is a need for the particles to be maintained in a sterile environment prior to actuation of the device. WO 94/24263 discloses for this purpose a particle cassette comprising a central annular ring having rupturable diaphragms sealed to each face so as to form a self contained sealed unit containing the particles to be injected. Upon actuation of the device, the diaphragms rupture allowing the particles initially contained between the diaphragms to be entrained in the gas flow and then delivered into the target.

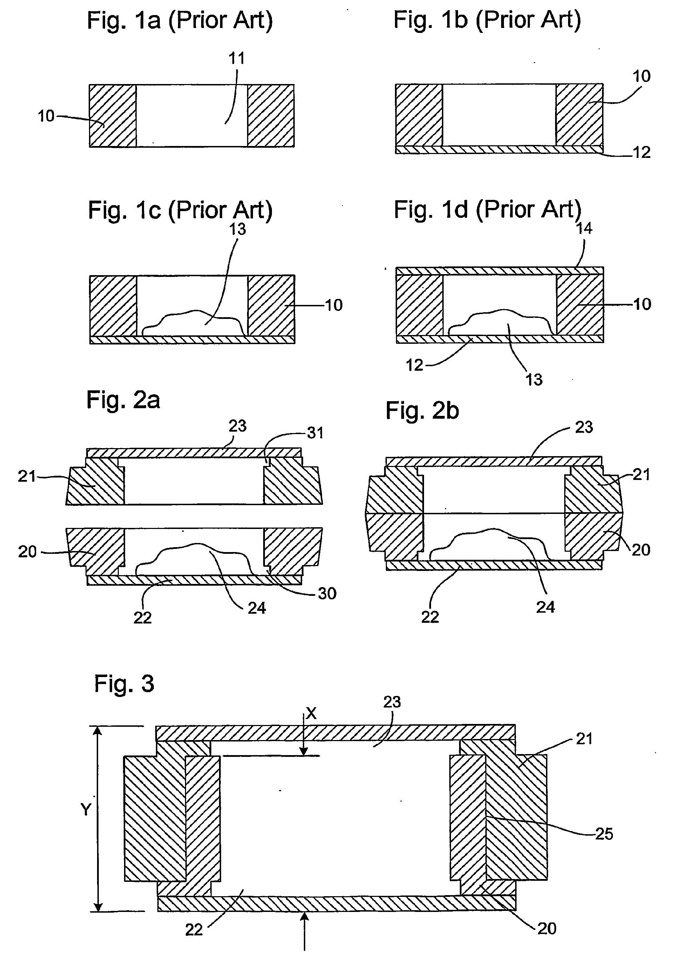

Figures 1a to 1d of the accompanying schematic drawings show steps in the manufacture of the particle cassette disclosed WO 94/24263. A substantially annular ring (10) is shown in axi-symmetric cross-section in Figure 1a. The ring has an open central section defining a chamber (11). In a first step of the manufacturing process a rupturable diaphragm (12) is sealed to the bottom face of the annular ring (10). This results in the construction shown in Figure 1b. Then, as shown in Figure 1c, particles (13) are supplied to the chamber (11). The particles (13) so supplied are those to be injected into the target. The last stage in manufacture comprises sealing a second membrane (14) onto the other face of the annular ring so as to seal the particles (13) within the central chamber (11). The cassette can then be handled while the particles are maintained in a sterile environment.

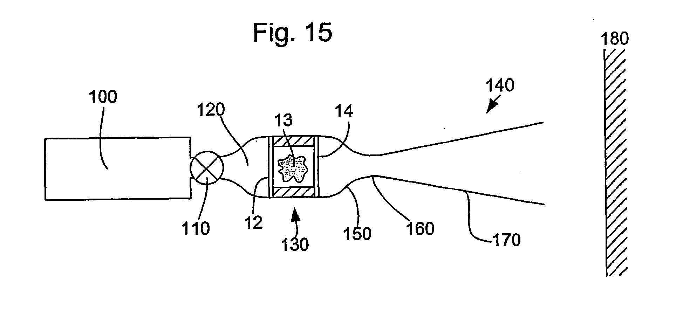

An exemplary needleless syringe is shown in Figure 15. As can be seen, the needleless syringe comprises a reservoir (100) of compressed gas connected to a valve (110). Downstream of the valve is an expansion chamber (120) the particle cassette (30) and a nozzle section (140). As shown in Figure 15 the nozzle section (140) comprises an upstream convergent portion (150) downstream of which is a throat (160) followed by a divergent portion (170). To activate the syringe, the valve (110) is actuated and gaseous pressure flows from the reservoir (100) into the expansion chamber (120). Pressure builds up behind the upstream membrane (12) until it reaches a sufficient value to cause the upstream membrane (12) to burst. The gas thereafter entrains the particles and the pressure once again builds up behind downstream membrane (14). Shortly afterwards, the downstream membrane (14) bursts so that the gas (with the particles (13) entrained) can be accelerated in the nozzle (140) and thence into a target (180).

WO 94/24263 discloses that the diaphragm should preferably be heat sealed to the faces of the annular ring. Heat sealing has been found to be a particularly easy and repeatable method of sealing the diaphragms to the ring of the cassette body.

It has been found that the above-mentioned configuration has a disadvantage associated with its manufacture, which may become especially deleterious when heat-sensitive particles (e.g. powdered drugs) are to be carried by the cassette. After the particles have been supplied to the chamber (11) formed by the annular ring (10) and the bottom membrane (12), the heat sealing of the top membrane (14) onto the ring (10) can sometimes result in a degradation of the particles. This degradation may take the form of melting, causing particle deformation, particle agglomeration and other undesirable physical and chemical changes in the product. Further, the melt may affect the therapeutic effect of the particles.

Accordingly, the present invention seeks to alleviate this problem by providing a particle cassette, a kit of parts and a method for the manufacture therefor in which the possibility of the particles melting during manufacture is much reduced.

In accordance with the first aspect of the present invention, there is provided a kit of parts for use in the manufacture of a particle cassette for a needleless syringe device according to claim 1. A document disclosing the features of the preamble of claim 1 is EP 0 951 917.

Thus, since the membranes are sealed to first and second cassette parts (possibly by heat sealing) before the particles are supplied to either of the cassette parts, there is a much reduced possibility of the membrane heat sealing process influencing the particle condition or composition. Further, the invention provides a quick and easy method of manufacturing a particle cassette and has these advantages over processes which do not involve heat sealing.

The first and second cassette parts are annular such that the second part is attachable concentrically around or inside the first cassette part. This allows the first cassette part to extend substantially along the whole width of the particle cassette making it easier to fill the first part with particles prior to attaching the second part.

To attach the first and second parts together, a variety of mechanisms may be used, including interference fits, friction fits, screw fits, detents and recesses, close tolerances, gluing etc. A snap fit may be provided for by arranging corresponding features on each of the first and second parts (for example a detent and a recess).

In order to ensure a consistent width of particle cassette, it is preferable to provide a seating face on each of the first and second parts, such a seating face providing a minimum possible width of particle cassette when assembled.

A tapered face on each of the first and second parts allows the parts to be brought together easily during assembly.

A third cassette part may be used to attach the first and second parts together. In a preferred embodiment, the third cassette part is inserted in an annular apace between the first and second cassette parts, to provide a secure attachment (or "locking"). The third part preferably has a third membrane to ensure sterility and may be provided with one or more protrusions to ensure an interference fit with the second cassette part.

In a second aspect of the invention, there is provided a particle cassette for a needleless syringe comprising an assembled kit according to the first aspect and particles provided in the chamber between the first and second membranes.

According to a third aspect of the present invention, there is provided a needleless syringe including the particle cassette of the second aspect of the invention.

According to a fourth aspect of the invention, there is provided a method of assembling a particle cassette according to the second aspect for a needleless syringe device, said method comprising:
(a) sealing a first rupturable membrane to a first cassette part;
(b) sealing a second rupturable membrane to a second cassette part;
(c) applying particles to said first cassette part;
(d) attaching said first and second cassette parts together so as to create a chamber confining said supplied particles between said first and second membranes.

The sealing of the membranes to the cassette parts independently from the steps of supplying particles to one of the cassette parts and attaching the cassette parts together ensures that the method used for sealing the membranes to the cassette parts does not unduly influence the quality of the particles in the cassette.

Preferably, attaching step (d) does not involve the application of any heat at all and it is preferably carried out at the same temperature as supplying step (c) to ensure that the particles are not affected by the step of attaching the first and second cassette parts together.

It is not essential that supplying step (c) is carried out after sealing step (b) since the second membrane may be sealed to the second cassette part after the particles have been supplied to the first cassette part.

Embodiments of the present invention will now be described, by way of example only, with reference to the accompanying schematic drawings, in which:-
Figures 1a to 1d are cross-sectional views showing stages in the manufacture of a particle cassette according to the prior art;
Figures 2a and 2b are cross-sectional views showing stages in the manufacture of a particle cassette not being part of the invention;
Figure 3 is a cross-sectional view showing an assembled particle cassette according to an embodiment of the present invention;
Figure 4 is a cross-sectional view showing an assembled particle cassette according to another embodiment of the present invention;
Figure 5 is a cross-sectional view showing an assembled particle cassette according to another embodiment of the present invention;
Figure 6 is a cross-sectional view showing an assembled particle cassette according to another of the present invention;
Figure 7 is a cross-sectional view of a second cassette part ;
Figure 8 is a cross-sectional view of a first cassette part
Figure 9 is a cross-sectional view along the line A-A shown in Figure 8;
Figure 10 is a cross-sectional view of a third cassette part
Figure 11 is a cross-sectional view of an assembled particle cassette with the parts of figures 7-10 according to another embodiment of the present invention;
Figure 12 shows two perspective views of a partially cut-away particle cassette according to the embodiment of figure 11;
Figure 13 is a set of three orthographic views of an assembled particle cassette according to another embodiment of the present invention;
Figure 14 is a set of three orthographic views of an assembled particle cassette according to another embodiment of the present invention; and
Figure 15 is a longitudinal cross-sectional view through a needleless syringe which may comprise the particle cassette of any one of the embodiments of the present invention.

In the drawings the components are not drawn to scale. The drawings are schematic for reasons of clarity. In reality the thickness of the rupturable diaphragms may be much less than that shown and/or the volume of particles may be so small as barely to be visible to the naked eye.

The present invention avoids the possibility of the heat sealing of the membranes affecting the particle quality by ensuring that the particles are confined within the chamber of the cassette by a step other than one involving heat sealing of one of the membranes. Thus, generally speaking, each of the embodiments of the present invention comprises a first cassette part (20) having a first membrane (22) sealed to a face and a second cassette part (21) having a second membrane (23) sealed to a face. Particles (24) are dispensed into the first cassette part and the second cassette part is then attached.

Figures 2a and 2b show an axi-symmetric cross-section of a particle cassette not being part of the present invention.

The first cassette part (20) comprises an annular ring having a first face (the upper face in Figure 2a) for attachment to a second cassette part (21), the other side of the annular ring having had a first membrane (22) sealed thereto. The second cassette part (21) is substantially identical to the first cassette part, having had a second membrane (23) sealed thereto. The first and second cassette parts, having the first and second membranes respectively, form a kit of parts for use in the manufacture of the particle cassette. The membranes are sealed to the external faces of the respective cassette parts. If heat sealing is used to attach the membranes then a recess (30,31) as shown in Figure 2a is useful because it allows for some plastic deformation as will be caused by heat sealing in general. In particular, the heat causes local expansion of the cassette material and the recesses (30,31) allow the cassette material to expand without affecting the designed gas flow path (eg by restricting the diameter of the flow path).

In order to manufacture the particle cassette, particles (24) are dispensed to the first cassette part (20) and the first (20) and second (21) cassette parts are attached together so as to create a closed chamber for the confinement of the particles (24) between the first (22) and second (23) membranes. The assembled particle cassette is shown in Figure 2b.

In use, the particle cassette is located in a needleless syringe device which may have the general construction, and method of operation, described in WO 94/24263 or WO 01/05455 . When located in the syringe device the device construction is advantageously such as to prevent the first cassette part (20) coming away from the second cassette part (21). However, in order to mitigate against further such detachment, the second cassette part (21) may be adhered to the first cassette part (20), for example by gluing or by taping around the external circumference of the particle cassette. This provides a sealed unit of particles which can be handled outside of the needleless syringe device with reduced possibility of particles escaping from between the two cassette halves.

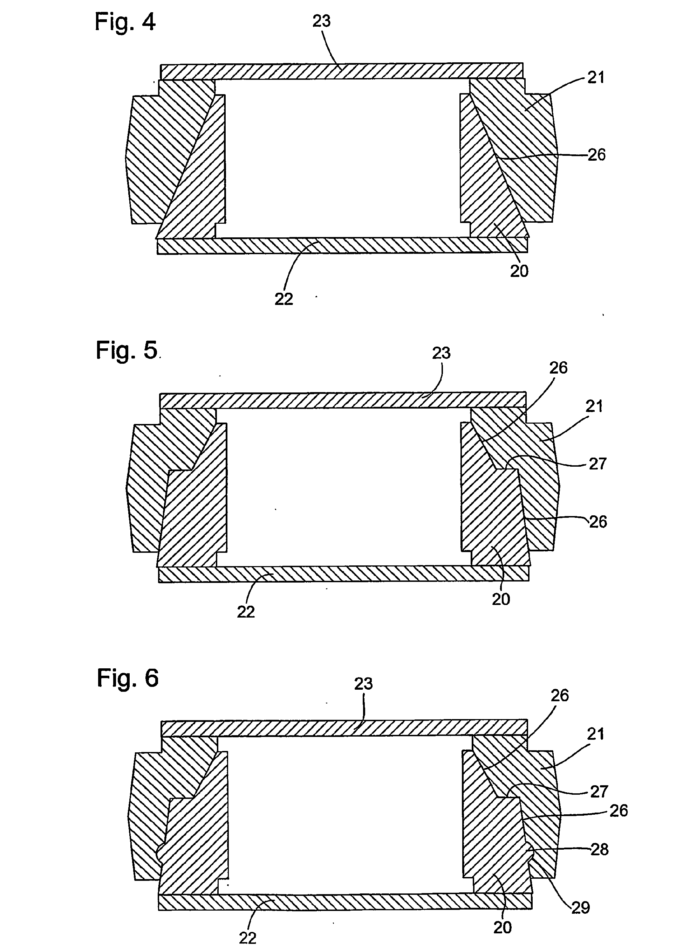

An embodiment of the invention is shown in Figure 3. The particles (24) are omitted from inside the particle cassette for the sake of clarity. Further, it is to be noted that the particle cassette shown in Figure 3 (and those shown in Figures 4 to 6 as well) will, prior to assembly, make up a kit of parts according to the first aspect of the present invention.

The first cassette part (20) is constituted by a substantially annular member which extends to a height X nearly equal to the height Y of the assembled particle cassette. This greatly facilitates the filling of the first cassette part (21) with particles (24) since a larger receptacle than is provided in the first embodiment can be used to receive particles (24). A further advantage is that the whole internal volume of the cassette can be used to hold particles. In contrast, the cassette of figures 2a and 2b can only be half filled with particles since the first cassette part (20) has a height equivalent only to approximately half the final height of the cassette.

The second cassette part (21) also has a substantially annular construction and is arranged to be attachable concentrically around the first cassette part (20). If necessary, adhesive can be used at the interface (25) to ensure that the first and second cassette parts do not detatch easily. More preferred, however, is that the cassette parts are attached by an interference fit, whereby the outer diameter of the engaging region of the first cassette part is slightly larger than the inner diameter of the engaging region of the second cassette part (21). In this way, the parts will naturally lock together due to the elastic strain established in each of the first and second cassette parts when the second part (21) is placed around the first part (20).

The embodiment shown above has advantages because it does not necessarily require an extra adhesive to be used (an interference fit is instead used) and because the first cassette part defines a larger receptacle area for receiving the particles (24). However, it may be difficult or fiddly to assemble, even if one or both of the engaging inner face of the second cassette part and the engaging outer face of the first cassette part is provided with a lead in taper to aid assembly. To overcome this problem, a cassette according to a further embodiment of the invention is provided. Such a cassette is shown in Figure 4.

In this embodiment of the present invention, the outer engaging face of the first cassette part (20) and the inner engaging face of the second cassette part (21) (the interface of which is denoted as (26) in Figure 4) are tapered so as to allow the second cassette part (21) to be easily placed over the first cassette part (20). Again, although gluing may be used to attach the parts, it is preferable that the first cassette part (20) has a larger outer diameter than the inner diameter of the second cassette part (21) at each point along the height of the cassette. This means that as the parts are brought close to the assembled position shown in Figure 4, some elastic strain is established in the parts to provide a snug fit. The parts are prevented from coming apart by friction along the tapered interface (26).

This embodiment of the invention thus has the advantage that it is easier to assemble the kit of parts than the previous embodiment of the invention. However, it has the potential disadvantage that the width of the cassette (ie the vertical dimension in Figure 4) may not necessarily be predicted before the parts are assembled. Depending on the force used to push the first (20) and second (21) cassette parts together, the width may vary over a certain range. For example, if the parts are pressed together very strongly, the width is likely to be less than if the parts are only lightly pressed together. Furthermore, the leading edge of the first cassette part (20) may damage the seal between the second membrane (23) and the second cassette part (21) if the two parts are pushed together too strongly.

To overcome this problem, a particle cassette as shown in a further embodiment of the invention is provided.

In this embodiment, a seating face (27) is provided to each of the first (20) and second (21) cassette parts. As is shown in Figure 5, once the cassette parts have been pushed together a certain amount, the seating face (27) prevents further movement in the cassette height direction (i.e. the vertical direction in Figure 5). This embodiment of the invention therefore allows the width dimension of the assembled particle cassette to be reliably ensured. The tapered sections (26) perform the same function as in the previous embodiment and the outer surfaces of the first cassette part (20) may again be made to have a slightly larger diameter than the inner surfaces of the second cassette part (21) so as to provide an interference fit. The steeper angle of the lower tapered section 26 provides for a better locked fit. The fairly uniform cross-section in the vicinity of the membranes allows for good heat dissipation during the heat sealing process, if used.

While an interference fit is suitable for a lot of purposes, it is often preferable that no elastic strains are built up in the first and second cassette parts, especially if they are to be re-used a lot of times. Another embodiment of the invention addresses this problem. Figure 6 shows an axi-symmetric cross-sectional view of this embodiment of the invention having parts generally similar to those shown in Figure 5.

The main difference is the provision on each of the first (20) and second (21) cassette parts of corresponding features (28, 29) which provide for a snap fit when the first and second cassette parts are brought together. In particular, the first cassette part (20) comprises a detent (28) located on one of external tapered surfaces (26). Correspondingly, the second cassette part (21) comprises a recess (29) on its respective tapered surface (26). Thus, when the first and second cassette parts are brought together, the detent (28) locates in the recess (29) to lock the two pieces together. During this attaching step, both the first and second cassette parts undergo a momentary elastic strain as the detent (28) engages in the recess (29) but, once assembled, the cassette part can be arranged so that there is little residual strain present. This means that there is no requirement to rely on an interference fit alone to maintain the first and second cassette parts in attachment. The provision of corresponding features also makes it more difficult for the first and second parts to be accidentally detached, providing a stronger lock between the first and second cassette parts and thus a more secure sealed environment for the particles. This is particularly useful when the cassette is to be subject to vibrations, such as those experienced during transportation. The embodiment shown in Figure 6 is considered to be the best mode of carrying out the invention.

This embodiment has a further advantage in that it provides an assembly that is tamper evident. Once assembled, it is very difficult to prise the cassette parts apart due to the taper lock and detent arrangement. Thus, the assembly could only be opened by destroying one of the membranes or using a very sharp tool to lever apart the parts, which in practice causes obvious deformation of the cassette parts.

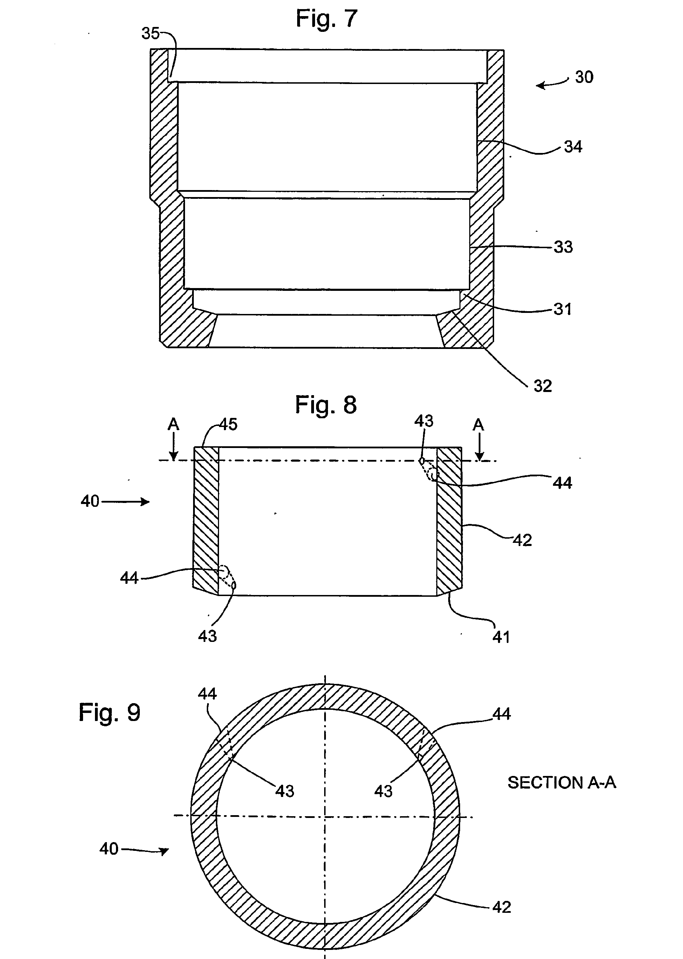

Figures 7 to 12 show a further embodiment of the invention. Unlike the previous embodiments, the particle cassette of this embodiment has three main component parts rather than two.

Figures 7 and 8 show in cross-sectional view second and first cassette parts respectively. The second cassette part (30) of Figure 7 is generally cylindrical in configuration having substantially vertical inside walls (33) and (34). The inside wall (33) has a slightly smaller diameter than inside wall (34) for reasons that will become apparent later. A shoulder portion (31) is provided adjacent a seating face (32). The shoulder portion (31) is designed to interact with the external surface of the first cassette part (40), described hereafter. A recess (35) is provided at the top end of the second cassette part (30) and this recess (35) aids in assembling the cassette.

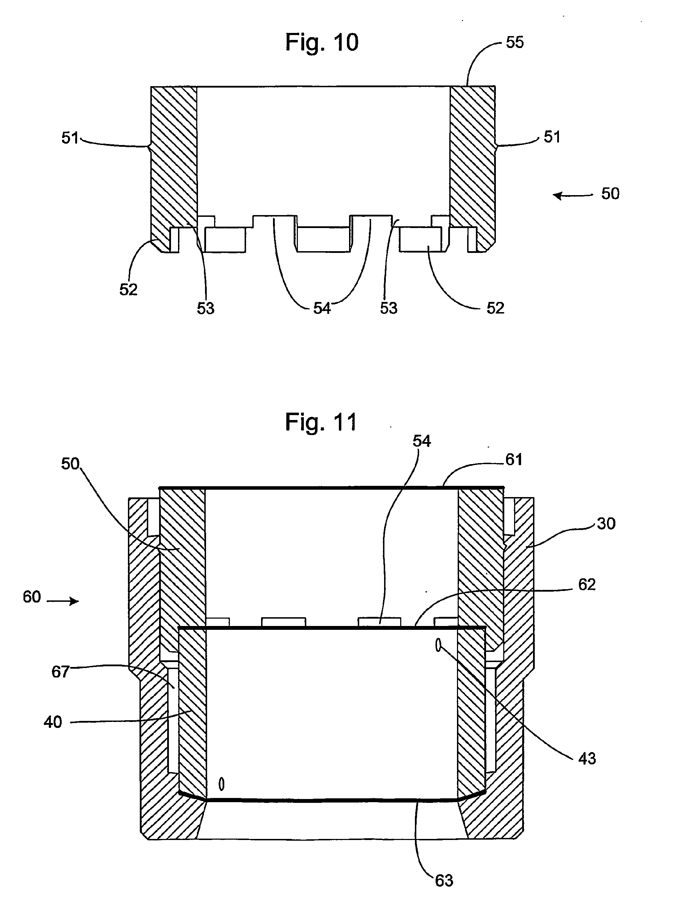

Figure 8 shows a first cassette part (40) according to this embodiment of the present invention. The first cassette part (40) comprises a substantially annular member defining within its confines a receptacle for receiving particles. The first cassette part (40) has generally cylindrical outer walls (42) and a slightly tapered seating face (41) at its bottom end. The seating face (41) is intended to rest against the seating face (32) of the second cassette part (30) during use. Furthermore, the shoulder (31) of the second cassette part (30) is intended to abut the outer surface (42) of the first cassette part to provide an interference fit. This configuration is shown in Figure 11.

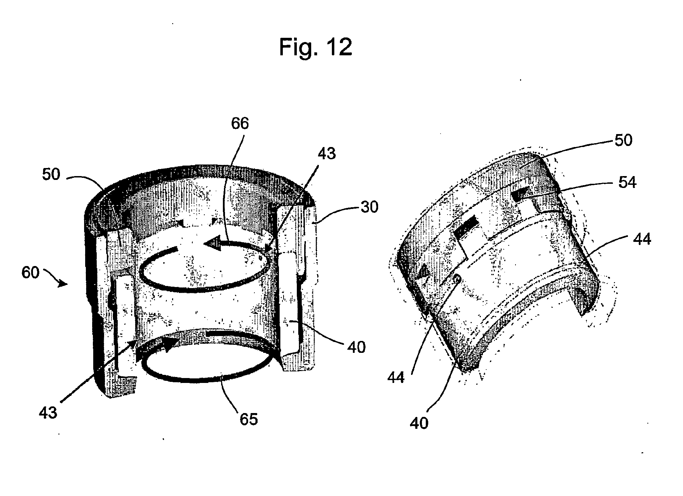

Referring back to Figures 8 and 9, there are openings (43) on the inner surface of the first cassette part (40) and openings (44) on the outer surface of the first cassette part (40). These openings are connected by transfer ducts such that gas surrounding the first cassette part (40) may enter the receptacle for receiving particles. The transfer ducts are substantially conical and are angled in three dimensions such that when gas flows therethrough a sonic jet is formed which tends to create a swirling gas movement inside the chamber confining the particles. The transfer ducts are angled such that the gas tends to impinge against the membranes located at either side of the first cassette part (40). Furthermore, the holes (43) are provided at different longitudinal ends of the first cassette part (40) and are directed in different directions such that a clockwise gas flow is established at one end of the particle confinement chamber and an anti-clockwise gas flow is established at the other end of the particle confinement chamber. This is illustrated in Figure 12 wherein the gas flows are referenced (65) and (66). As is clear from Figure 9, the transfer ducts are provided on the same lateral side of the first cassette part (i.e. above the centre line shown in Figure 9), and this has been found to provide for good fluidization of the particles when gas pressure is introduced to the openings (44) and a flow of gas is established through the transfer ducts. However, other configurations of transfer duct in the side walls of the first particle cassette part (40) can provide good results and it is not generally essential for the present invention that the transfer ducts have the specific form shown in Figures 8 and 9.

Figure 10 shows a third particle cassette part in accordance with this embodiment of the present invention.

The third cassette part (50), in common with the first and second cassette parts, has generally cylindrical inner and outer walls forming an annular-shaped member. One or more protrusions (51) may be formed on the outer walls and these are intended to provide an interference fit against the inner wall (34) of the second cassette part (30), when the particle cassette is assembled. The lower end of the third cassette part (50) has a number of formations (52) around the circumference. The formations (52) are stepped and are designed such that the top part (53) of the formations (52) abuts the top surface of the first cassette part (40) when assembled, as shown in Figure 11. The formations are spaced apart by vent holes (54) that are formed such that gas may pass through the vent holes (54) when the first and third cassette parts are attached together. The formations (52) are shaped so as to grip, by means of friction, or interference, the top part of the first cassette part (40).

The particle cassette according to this embodiment of the invention takes the form shown in Figure 11 when assembled. In this embodiment, there are three membranes (61, 62, 63) of which one membrane (61) is relatively thin with a fairly low bursting pressure and is designed to keep the unit sterile in use.

To assemble the particle cassette of this embodiment, a first membrane (62) is heat sealed or bonded to the upper edge of the first cassette part (40). Similarly, the second membranes (63) is heat sealed or bonded to the seating face (32) of the second cassette part (30). The third membrane (61) is heat sealed or bonded to the upper face of the third cassette part (50). The first membrane and first cassette part thus define a receptacle in which the particles may be contained. The openings (43) are very small such that it is very difficult for the particles to pass out of the chamber once inside. Once the particles have been supplied to the chamber of the first cassette part (40), the first cassette part (40) is brought together with the second cassette part (30) with the leading edge of the first cassette part engaging the shoulders (31) of the second cassette part. The first cassette part (40) is pushed home until the seating face (41) of the first cassette part abuts the seating face (32) of the second cassette part (with the second membrane (63) between the two seating faces). In this configuration, the particles are trapped between the first and second membranes. The third cassette part (50) having the third membrane (61) thereon is then pushed in so that the formations (52) slide into the annular gap created between the first and second cassette parts. Interference and/or friction ensure that this movement firmly secures the first and second parts together and effectively "locks" the cassette. It will be appreciated that it is quite difficult to remove the third cassette part once it is installed, especially if the top face (55) of the third cassette part is dimensioned so as to be flush with the top face of the second cassette part when assembled (this is not shown in Figure 11 however).

The membrane (61) ensures that the particles inside the cassette may not come into contact with any external particles or gases and thus the membrane (61) ensures the sterility of the cassette.

In use, the cassette is inserted into a needleless syringe and gas pressure is supplied to the third membrane (61). The membrane (61) bursts quite easily and gas enters the internal space defined by the third cassette part. Gas is able to flow through the vents (54) and into the annular space (67) between the first cassette part and the second cassette part. From there, gas may pass through the transfer ducts and out through the openings (43) into the particle containment chamber. The jets of gas so formed cause the particles to be fluidized and mixed. Following that, the upstream membrane (62) bursts and the particles are entrained in the bulk of the gas flow followed by the bursting of the downstream membrane (63) shortly thereafter. In this way, the particle cassette of the last embodiment provides for pre-mixing and fluidizing of the particles whilst still overcoming the problem that heat sealing of membranes can damage particles when a single piece cassette is used.

The concepts described in relation to the previous embodiments may also be applied to the last embodiment in the same way. For example, a snap fit may be provided for if detents and corresponding recesses are provided on either the first and second, first and third or second and third cassette parts respectively.

Heat sealing or adhesive is not necessary in the last embodiment and the first and second membranes may be sealed against the first and second cassette parts respectively due to the tight fit between the various cassette parts. For example, the first membrane (62) may be sealed by virtue of being trapped between the first and third cassette parts. Similarly, the second membrane (63) may be trapped between the first and second cassette parts, with no special heat sealing or adhesive step being required.

As can be seen from Figure 15 in particular, the embodiments are usually used in a configuration such that the particle cassette is located downstream of an expansion chamber (120) in the needleless syringe. The syringe is therefore complex to assemble.

To overcome this problem, a particle cassette as shown in two further embodiments of the invention is provided. The particle cassette itself has a built in expansion chamber (120) meaning that the syringe has less components overall. This results in a decrease in manufacturing time and complexity.

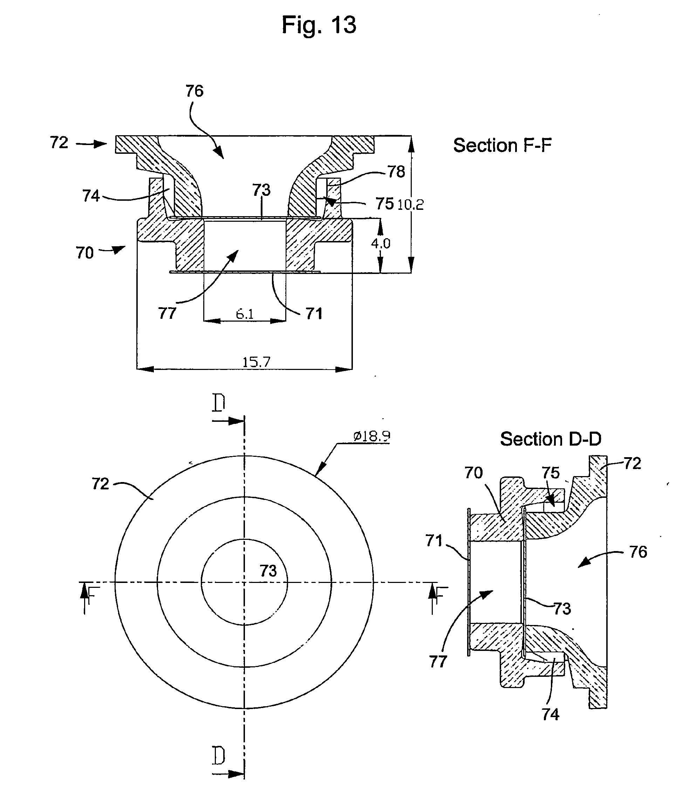

Another embodiment of the invention is shown in Figure 13. In this embodiment, the first cassette part (70) has heat sealed to a lower edge a membrane (71). Particles are then located in chamber (77) formed by the inner side walls of the first cassette part (70) and the membrane (71). The second cassette part (72) can then be placed on top of the first cassette part so as to seal off chamber (77). As can be seen from Figure 13, the second cassette part (72) comprises a substantially annular member having a membrane (73) attached (eg. by heat sealing) to its lower edge. The annular second member fits inside part of the annular first member. The second member is locked against the first member by means of ribs (74) on the second member which locate in spaces (75) created between the first and second members during assembly. As can be seen, the ribs (74) have a tapered front edge to aid assembly and are made so as to provide a snug interference fit with the inside annular surface (78) of the first member.

It will also be apparent from Figure 13 that this embodiment comprises an open chamber (76) that is arranged to open outwardly of the assembled cassette. The chamber (76) lies above (i.e. upstream of) the membrane (73) which separates it from the particle confinement chamber (77). When the cassette is assembled in the finished syringe, the chamber (76) performs the function of the expansion chamber (120) shown in Figure 15. Please note that Figure 15 is schematic so that, even though Figure 15 shows an expansion chamber (120) which diverges in the downstream direction, the expansion chamber provided by chamber (76) of the particle cassette of the seventh embodiment converges in the downstream direction and this results in a needleless syringe which works adequately.

It can be seen that the particle cassette of this embodiment retains the advantages of the previous embodiments in that the particles can be located in chamber (77) and the two cassette parts pressed together to confine the particles in a chamber, without the need to subject the particles to the heating that often accompanies sealing the membranes to the cassette parts. Furthermore, this embodiment integrates part of the needleless syringe, viz, the expansion chamber (120), with the particle cassette, thereby reducing the number of components and the manufacturing time and expense.

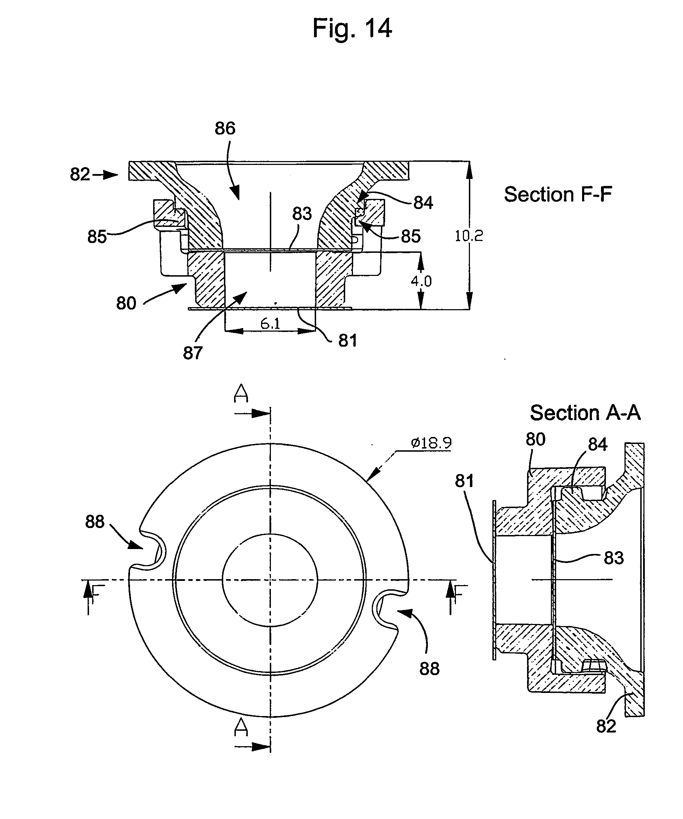

Another embodiment of the invention is shown in Figure 14. This embodiment has a construction very similar to that of the previous embodiment, save for the particular manner in which the first cassette part (80) is connected to the second cassette part (82). Whilst in the previous embodiment the two parts are pushed together to form an interference fit, the two parts are screwed together in this embodiment by means of threads (84 and 85). A thread (84) is located on the outer surface of the second cassette part (82) and is designed to mate with a corresponding thread (85) located on the inner surface of first cassette part (80).

It will be apparent from both Figures 13 and 14 that the first cassette part (70 or 80) contains annular longitudinal protrusions which extend beyond the plane of the upstream membrane (73 or 83) in use. These protrusions allow the first part (70 or 80) to be connected to the respective second part (72 or 82) either by means of an interference fit as shown in Figure 13, or by means of a screw thread as shown in Figure 14.

The screw thread is preferably relatively coarse such that the first and second cassette parts can be connected together with relatively few turns. Preferably, less than one turn of relative movement is necessary, more preferably 180° or less of relative rotation. In the most preferred embodiment, a range of movement between 45° and 120°, preferably 90° is sufficient to attach the first and second cassette parts together.

Of course, the various means for attaching the cassette parts shown in the previous embodiments may be combined with the features of the letter embodiment, for example, seating faces, tapers and detents which provide a "snap-fit" can all be used in combination with the screw thread attachment. Furthermore, a third cassette part may be used in the last two embodiments to lock the first and second cassette parts together.

It will be noted from Figure 14 that two indents (88) are provided on the outer circumferential surface of the second cassette part (82). These indents are designed to receive a tool which assists in applying a torque to the second cassette part (82) when it is screwed to the first cassette part (80). Similar indents may also be provided on the first cassette part (80) if required.

The screw thread design of the last embodiment has been found to give a more reproducible face seal between the mating surfaces of the first and second cassette parts. This helps to prevents egress of payload from the chamber (87) inside the assembled cassette, and gives a more consistent barrier to potential biological contamination. Furthermore, the integration of the expansion chamber (120) with the particle cassette makes the overall dimensions of the cassette larger than in the prior art which facilitates handling and manipulation during manufacture of the needleless syringe comprising the casssette.

It is envisaged that other embodiments falling within the scope of the present claims can be provided by using combinations of the various attaching methods disclosed in the described embodiments herein. The type of attaching means to be used depends on the circumstances. For example, there are occasions when a "snap-fit" action is not suitable because the snapping provides a vibrational jolt to the cassette assembly which may damage particularly fragile particles. In such cases, an interference fit or screw fit is preferred.

For each of the embodiments, the materials used to manufacture the cassette parts and the membranes may be conventional, for example, the membranes may be Mylar as disclosed in WO 94/24263 and the first and second cassette parts are preferably manufactured from a plastics material, using injection moulding for example. Both the membranes and cassette parts may be made from polycarbonate such as Evaxone 260 (EVA) polymer. If heat sealing is used, a temperature of 110°C and pressure of 760 kPa (110 psi) for 1.5 seconds has been found to be acceptable.

The cassette is suitable for any type of particle that one intends to deliver, including powdered drugs (therapeutics, medicaments, vaccines, anaesthetics, analgesics, and the like), diagnostic particles (whether inert or comprising an active ingredient), and carrier particles coated with peptides, proteins or genetic material.

## Claims

1. A kit of parts for use in the manufacture of a particle cassette for a needleless syringe device, said kit comprising:
a first cassette (20) part having a first rupturable membrane (22) sealed thereto; and
a second cassette part (21) having a second rupturable membrane (23) sealed thereto; said first and second cassette parts being arranged to be attachable together so as to create a chamber for the confinement of particles (24) between said first and second membranes;
wherein said first (20) and second (21) cassette parts are substantially annular and said first cassette part defines a receptacle for receiving particles;
**characterised in that:**
said second cassette part (21) is attachable concentrically around or inside said first cassette part (20).

2. A kit according to claim 1, wherein said first (20) and second (21) cassette parts are arranged to be attached together by an interference fit.

3. A kit according to any one of the preceding claims, wherein said first and second cassette parts each comprise corresponding features (28,29) which provide for a snap fit when said first and second cassette parts are attached together.

4. A kit according to claim 3, wherein said corresponding features comprise a detent (28) and a recess (29).

5. A kit according to any one of the preceding claims, wherein said first and second cassette parts each comprise a seating face (27) which ensures the attainment of a predetermined dimension of said cassette in the direction in which said first (20) and second (21) parts are attachable together.

6. A kit according to any one of the preceding claims, wherein said first and second cassette parts each comprise a tapered face (26) which are arranged to contact one another fully when said cassette is properly assembled.

7. A kit according to claim 3, wherein said corresponding features are provided on tapered faces which are arranged to contact one another fully when said cassette is properly assembled.

8. A kit according to any one of the preceding claims, further comprising a third cassette part (50) for locking together said first (40) and second (30) cassette parts.

9. A kit according to claim 8, wherein said third cassette part (50) has a third membrane (61) sealed thereto.

10. A kit according to claim 8 or 9, wherein said third cassette part (50) comprises a protrusion (51) for creating an interference fit with said second cassette part (30).

11. A kit according to any one of the preceding claims, wherein said first and second cassette parts are shaped so as to be fitted together leaving an annular gap (67) around said first cassette part (40).

12. A kit according to claim 8, 9 or 10, wherein said first and second cassette parts are shaped so as to be fitted together leaving an annular gap (67) around said first cassette part (40) and said third cassette part (50) has a portion (52) which inserts into said annular gap (67) so as to lock said first, second and third cassette parts together.

13. A kit according to any one of the preceding claims, wherein said first cassette part (40) comprises at least one transfer duct (43) for supplying gas to said particle confinement chamber.

14. A kit according to claim 8, 9, 10 or 12, wherein said third cassette part (50) comprises gas ports (54) for supplying gas to an annular space (67) around said first cassette part.

15. A kit according to any one of the preceding claims, wherein said first and second cassette parts are arranged to be attached by a screw thread (84,85).

16. A kit according to claim 15, wherein said screw thread (84,85) has a coarse pitch such that the first and second cassette parts can be attached together by a relative rotation of 180° or less.

17. A kit according to one of the preceding claims, wherein one of said first and second cassette parts (82) comprises an open chamber (86) that is arranged to open outwardly of the assembled cassette when the first and second cassette parts are attached together.

18. A particle cassette for a needleless syringe comprising:
an assembled kit of the parts claimed in any one of claims 1 to 17; and
particles (24) provided in said chamber between said first and second membranes.

19. A particle cassette according to claim 18, wherein said particles comprise powdered drug.

20. A needleless syringe including the particle cassette of claim 19.

21. A method of assembling a particle cassette for a needleless syringe device according to anyone of claims 18 and 19, said method comprising:
(a) sealing a first rupturable membrane (22) to a first cassette part (20);
(b) sealing a second rupturable membrane (23) to a second cassette part (21);
(c) supplying particles (24) to said first cassette part (20); and
(d) attaching said first and second cassette parts together so as to create a chamber confining said supplied particles (24) between said first and second membranes.

22. A method according to claim 21, wherein said sealing steps (a) and (b) comprise heat sealing.

23. A method according to claim 21 or 22, wherein attaching step (d) does not involve the application of heat.

24. A method according to claim 21, 22 or 23, wherein attaching step (d) is carried out at the same temperature as supplying step (c).

25. A method according to any one of claims 21 to 24, wherein said first cassette part (20) is substantially annular and defines a receptacle, said particles being supplied to said receptacle in step (c).

26. A method according to any one of claims 21 to 25, wherein said second cassette part (21) is substantially annular and step (d) comprises fitting said second cassette part concentrically around or inside said first cassette part.

27. A method according to any one of claims 21 to 26, wherein step (d) comprises establishing an interference fit between said first and second cassette parts.

28. A method according to any one of claims 21 to 27, wherein said first and second cassette parts each comprise corresponding features (28,29) which provide for a snap fit when said first and second cassette parts are attached and step (d) comprises associating said corresponding features to snap said second cassette part onto said first cassette part.

29. A method according to claim 28, wherein said corresponding features comprise a detent (28) and a recess (29) and step (d) comprises locating said detent in said recess.

30. A method according to any one of claims 21 to 29, wherein sealing steps (a) and (b) comprise sealing said first and second membranes around their periphery to substantially annular first and second cassette parts respectively.

31. A method according to any one of claims 21 to 30, wherein step (c) is carried out before step (b).

32. A method according to any one of claims 21 to 31, wherein said first and second cassette parts each comprise a seating face (27) substantially perpendicular to the direction in which the parts are moved to be attached and step (d) further comprises the contacting of said respective seating faces.

33. A method according to any one of claims 21 to 32, wherein said first and second cassette parts each comprise a tapered face (26) at an acute angle to the direction in which the parts are moved to be attached and step (d) further comprises the contacting of said respective tapered faces.

34. A method according to any one of claims 21 to 33, wherein said attaching step (d) comprises introducing a third cassette part (50) so as to attach said first and second cassette parts together.

35. A method according to claim 34, further comprising sealing a third rupturable membrane (61) to said third cassette part (50) before employing said third cassette part.

36. A method according to any one of claims 21 to 35, wherein step (d) comprises rotating the first and second cassette parts relative to one another so as to screw them together.

37. A method according to claim 36, wherein said relative rotation is less than 180°.

## Patentansprüche

1. Kit zur Verwendung bei der Herstellung einer Partikelkassette für eine nadellose Spritzenvorrichtung, wobei der Kit umfasst:
einen ersten Kassetten (20)-Teil mit einer ersten zerreißbaren Membran (22), die damit versiegelt ist; und
einen zweiten Kassetten (21)-Teil mit einer zweiten zerreißbaren Membran (23), die damit versiegelt ist; wobei der erste und der zweite Kassenteil so angeordnet sind, dass sie aneinander anfügbar sind, so dass sie eine Kammer zum Einschluss von Partikeln (24) zwischen der ersten und der zweiten Membran bilden;
wobei der erste (20) und der zweite (21) Kassettenteil im Wesentlichen ringförmig sind und der erste Kassettenteil ein Aufnahmegefäß zur Aufnahme von Partikeln definiert, **dadurch**
**gekennzeichnet, dass:**
der zweite Kassettenteil (21) konzentrisch um oder innerhalb des ersten Kassettenteils (20) anfügbar ist.

2. Kit gemäß Anspruch 1, wobei der erste (20) und der zweite (21) Kassettenteil so angeordnet sind, dass sie durch eine Presspassung aneinander angefügt werden.

3. Kit gemäß einem der vorhergehenden Ansprüche, wobei der erste und der zweite Kassettenteil jeweils entsprechende Merkmale (28, 29) umfassen, die eine Schnappverbindung bereitstellen, wenn der erste und der zweite Kassettenteil aneinander gefügt werden.

4. Kit gemäß Anspruch 3, wobei die sich entsprechenden Merkmale eine Rastung (28) und eine Ausnehmung (29) umfassen.

5. Kit gemäß einem der vorhergehenden Ansprüche, wobei der erste und der zweite Kassettenteil jeweils eine Auflagefläche (27) umfassen, die die Erlangung einer zuvor festgelegten Dimension der Kassette in der Richtung, in der der erste (20) und der zweite (21) Teil aneinander anfügbar sind, sicherstellt.

6. Kit gemäß einem der vorhergehenden Ansprüche, wobei der erste und der zweite Kassettenteil jeweils eine konische Fläche (26) umfassen, die so angeordnet sind, dass sie vollständig miteinander in Kontakt stehen, wenn die Kassette richtig zusammengesetzt ist.

7. Kit gemäß Anspruch 3, wobei die sich entsprechenden Merkmale auf konischen Flächen bereitgestellt werden, die so angeordnet sind, dass sie vollständig miteinander in Kontakt stehen, wenn die Kassette richtig zusammengesetzt ist.

8. Kit gemäß einem der vorhergehenden Ansprüche, weiterhin umfassend einen dritten Kassettenteil (50) zum Arretieren des ersten (40) und des zweiten (30) Kassettenteils miteinander.

9. Kit gemäß Anspruch 8, wobei der dritte Kassettenteil (50) eine dritte Membran (61), die damit versiegelt ist, aufweist.

10. Kit gemäß Anspruch 8 oder 9, wobei der dritte Kassettenteil (50) einen Vorsprung (51) zum Erzeugen einer Presspassung mit dem zweiten Kassettenteil (30) umfasst.

11. Kit gemäß einem der vorhergehenden Ansprüche, wobei der erste und der zweite Kassettenteil so geformt sind, dass sie aneinander angepasst sind, wobei eine ringförmige Lücke (67) um den ersten Kassettenteil (40) belassen wird.

12. Kit gemäß Anspruch 8, 9 oder 10, wobei der erste und der zweite Kassettenteil so geformt sind, dass sie aneinander angepasst sind, wobei eine ringförmige Lücke (67) um den ersten Kassettenteil (40) belassen wird, und der dritte Kassettenteil (50) einen Teil (52) aufweist, der sich in die ringförmige Lücke (67) so einfügt, dass der erste, der zweite und der dritte Kassettenteil miteinander arretiert werden.

13. Kit gemäß einem der vorhergehenden Ansprüche, wobei der erste Kassettenteil (40) mindestens einen Übertragungskanal (43) zum Zuführen von Gas an die Partikeleinschlusskammer umfasst.

14. Kit gemäß Anspruch 8, 9, 10 oder 12, wobei der dritte Kassettenteil (50) Gasanschlüsse (54) zum Zuführen von Gas an einen ringförmigen Raum (67) um den ersten Kassettenteil umfasst.

15. Kit gemäß einem der vorhergehenden Ansprüche, wobei der erste und der zweite Kassettenteil so angeordnet sind, dass sie durch eine Schraubverbindung (84, 85) aneinandergefügt werden.

16. Kit gemäß Anspruch 15, wobei die Schraubverbindung (84, 85) ein Grobgewinde aufweist, so dass der erste und der zweite Kassettenteil durch eine relative Drehung von 180° oder weniger aneinander anfügbar sind.

17. Kit gemäß einem der vorhergehenden Ansprüche, wobei einer des ersten und des zweiten Kassettenteils (82) eine offene Kammer (86) umfasst, die so angeordnet ist, dass sie sich nach außen von der zusammengesetzten Kassette öffnet, wenn der erste und der zweite Kassettenteil aneinandergefügt sind.

18. Partikelkassette für eine nadellose Spritze, umfassend einen zusammengesetzten Kit, beansprucht in einem der Ansprüche 1 bis 17, und Partikel (24), die in der Kammer zwischen der ersten und der zweiten Membran bereitgestellt werden.

19. Partikelkassette nach Anspruch 18, wobei die Partikel einen pulverisierten Wirkstoff umfassen.

20. Nadellose Spritze, einschließlich der Partikelkassette nach Anspruch 19.

21. Verfahren zum Zusammensetzen einer Partikelkassette für eine nadellose Spritzenvorrichtung gemäß einem der Ansprüche 18 und 19, umfassend:
(a) Versiegeln einer ersten zerreißbaren Membran (22) mit einem ersten Kassettenteil (20);
(b) Versiegeln einer zweiten zerreißbaren Membran (23) mit einem zweiten Kassettenteil (21);
(c) Zuführen von Partikeln (24) an den ersten Kassettenteil (20); und
(d) Aneinanderfügen des ersten und des zweiten Kassettenteils, so dass eine Kammer erzeugt wird, die die zugeführten Partikel (24) zwischen der ersten und der zweiten Membran umschließt.

22. Verfahren gemäß Anspruch 21, wobei die Versiegelungsstufen (a) und (b) Heißsiegeln umfassen.

23. Verfahren gemäß Anspruch 21 oder 22, wobei die Anfügungsstufe (d) nicht die Anwendung von Wärme umfasst.

24. Verfahren gemäß Anspruch 21, 22 oder 23, wobei die Anfügungsstufe (d) bei der gleichen Temperatur durchgeführt wird wie die Zuführungsstufe (c).

25. Verfahren gemäß einem der Ansprüche 21 bis 24, wobei der erste Kassettenteil (20) im Wesentlichen ringförmig ist und ein Aufnahmegefäß definiert, wobei diesem Aufnahmegefäß in Stufe (c) Partikel zugeführt werden.

26. Verfahren gemäß einem der Ansprüche 21 bis 25, wobei der zweite Kassettenteil (21) im Wesentlichen ringförmig ist und Stufe (d) das passende Anbringen des zweiten Kassettenteils konzentrisch um oder innerhalb des ersten Kassettenteils umfasst.

27. Verfahren gemäß einem der Ansprüche 21 bis 26, wobei Stufe (d) das Einrichten einer Presspassung zwischen dem ersten und dem zweiten Kassettenteil umfasst.

28. Verfahren gemäß einem der Ansprüche 21 bis 27, wobei der erste und der zweite Kassettenteil jeweils sich entsprechende Merkmale (28, 29) umfassen, die eine Schnappverbindung bereitstellen, wenn der erste und der zweite Kassettenteil aneinandergefügt werden, und Stufe (d) das Verbinden der sich entsprechenden Merkmale umfasst, um den zweiten Kassettenteil auf dem ersten Kassettenteil einzurasten.

29. Verfahren gemäß Anspruch 28, wobei die sich entsprechenden Merkmale eine Rastung (28) und eine Ausnehmung (29) umfassen und Stufe (d) das Lokalisieren der Rastung in der Ausnehmung umfasst.

30. Verfahren gemäß einem der Ansprüche 21 bis 29, wobei die Versiegelungsstufen (a) und (b) das Versiegeln der ersten und der zweiten Membran um ihren Umfang mit dem im Wesentlichen ringförmigen ersten bzw. zweiten Kassettenteil umfassen.

31. Verfahren gemäß einem der Ansprüche 21 bis 30, wobei Stufe (c) vor Stufe (b) durchgeführt wird.

32. Verfahren gemäß einem der Ansprüche 21 bis 31, wobei der erste und der zweite Kassettenteil jeweils eine Auflagefläche (27) umfassen, die im Wesentlichen senkrecht zu der Richtung ist, in welcher die Teile bewegt werden, die angefügt werden sollen, und Stufe (d) weiterhin das Inkontaktbringen der entsprechenden Auflageflächen umfasst.

33. Verfahren gemäß einem der Ansprüche 21 bis 32, wobei der erste und der zweite Kassettenteil jeweils eine konische Fläche (26) mit einem spitzen Winkel in der Richtung umfassen, in welcher die Teile bewegt werden, die angefügt werden sollen, und Stufe (d) weiter das Inkontaktbringen der entsprechenden konischen Flächen umfasst.

34. Verfahren gemäß einem der Ansprüche 21 bis 33, wobei die Anfügungsstufe (d) das Einführen eines dritten Kassettenteils (50) umfasst, so dass der erste und der zweite Kassettenteil aneinandergefügt werden.

35. Verfahren gemäß Anspruch 34, weiterhin umfassend das Versiegeln einer dritten zerreißbaren Membran (61) mit dem dritten Kassettenteil (50), bevor der dritte Kassettenteil verwendet wird.

36. Verfahren gemäß einem der Ansprüche 21 bis 35, wobei Stufe (d) das Drehen des ersten und des zweiten Kassettenteils relativ zueinander umfasst, so dass sie zusammengeschraubt werden.

37. Verfahren gemäß Anspruch 26, wobei die relative Drehung weniger als 180° beträgt.

## Revendications

1. Kit de pièces destiné à être utilisé dans la fabrication d'une cassette à particules pour un dispositif à seringue sans aiguille, ledit kit comportant :
une première partie de cassette (20) ayant une première membrane (22) pouvant être rompue, qui lui est scellée ; et
une seconde partie de cassette (21) ayant une seconde membrane (23) qui peut être rompue, qui lui est scellée ;
lesdites première et seconde parties de cassette étant agencées de façon à pouvoir être attachées l'une à l'autre afin de créer une chambre destinée au confinement de particules (24) entre lesdites première et seconde membranes ;
dans lequel lesdites première (20) et seconde (21) parties de cassette sont sensiblement annulaires et ladite première partie de cassette définit un réceptacle destiné à recevoir des particules ;
**caractérisé en ce que :**
ladite seconde partie de cassette (21) peut être attachée concentriquement autour ou à l'intérieur de ladite première partie de cassette (20).

2. Kit selon la revendication 1, dans lequel lesdites première (20) et seconde (21) parties de cassette sont agencées de façon à être attachées l'une à l'autre par un ajustement serré.

3. Kit selon l'une des revendications précédentes, dans lequel lesdites première et seconde parties de cassette comprennent chacune des détails correspondants (28, 29) qui réalisent un assemblage par encliquetage lorsque lesdites première et seconde parties de cassette sont attachées l'une à l'autre.

4. Kit selon la revendication 3, dans lequel lesdits détails correspondants comprennent un bossage (28) et un évidement (29).

5. Kit selon l'une des revendications précédentes, dans lequel lesdites première et seconde parties de cassette comprennent chacune une face d'appui (27) qui assure l'obtention d'une dimension prédéterminée de ladite cassette dans la direction dans laquelle lesdites première (20) et seconde (21) parties peuvent être attachées l'une à l'autre.

6. Kit selon l'une des revendications précédentes, dans lequel lesdites première et seconde parties de cassette comprennent chacune une face oblique (26), lesquelles faces (26) sont agencées de façon à entrer en contact entre elles complètement lorsque ladite cassette est convenablement assemblée.

7. Kit selon la revendication 3, dans lequel lesdits détails correspondants sont situés sur des faces obliques qui sont agencées de façon à entrer en contact entre elles complètement lorsque ladite cassette est convenablement assemblée.

8. Kit selon l'une des revendications précédentes, comportant en outre une troisième partie de cassette (50) destinée à verrouiller entre elles lesdites première (40) et seconde (30) parties de cassette.

9. Kit selon la revendication 8, dans lequel ladite troisième partie de cassette (50) comporte une troisième membrane (61) qui lui est scellée.

10. Kit selon la revendication 8 ou 9, dans lequel ladite troisième partie de cassette (50) comporte une saillie (51) destinée à créer un ajustement serré avec ladite deuxième partie de cassette (30).

11. Kit selon l'une des revendications précédentes, dans lequel lesdites première et deuxième parties de cassette sont configurées de façon à être ajustées l'une à l'autre en laissant un intervalle annulaire (67) autour de ladite première partie de cassette (40).

12. Kit selon la revendication 8, 9 ou 10, dans lequel lesdites première et deuxième parties de cassette sont configurées de façon à être ajustées l'une à l'autre en laissant un espace annulaire (67) autour de ladite première partie de cassette (40) et ladite troisième partie de cassette (50) comporte une portion (52) qui s'insère dans ledit intervalle annulaire (67) afin de verrouiller entre elles lesdites première, deuxième et troisième parties de cassette.

13. Kit selon l'une des revendications précédentes, dans lequel ladite première partie de cassette (40) comporte au moins un conduit (43) de transfert destiné à amener du gaz à ladite chambre de confinement de particules.

14. Kit selon la revendication 8, 9, 10 ou 12, dans lequel ladite troisième partie de cassette (50) présente des orifices (54) de gaz destinés à amener un gaz à un espace annulaire (67) autour de ladite première partie de cassette.

15. Kit selon l'une des revendications précédentes, dans lequel lesdites première et deuxième parties de cassette sont agencées de façon à être attachées par un filet de vis (84, 85).

16. Kit selon la revendication 15, dans lequel ledit filet de vis (84, 85) a un pas grossier tel que les première et deuxième parties de cassette peuvent être attachées l'une à l'autre par une rotation relative de 180° ou moins.

17. Kit selon l'une des revendications précédentes, dans lequel l'une desdites première et deuxième parties de cassette (82) présente une chambre ouverte (86) qui est agencée pour s'ouvrir vers l'extérieur de la cassette assemblée lorsque les première et deuxième parties de cassette sont attachées l'une à l'autre.

18. Cassette à particules pour une seringue sans aiguille comportant :
un kit assemblé des pièces revendiquées dans l'une quelconque des revendications 1 à 17 ; et
des particules (24) se trouvant dans ladite chambre entre lesdites première et seconde membranes.

19. Cassette à particules selon la revendication 18, dans laquelle lesdites particules comprennent un médicament en poudre.

20. Seringue sans aiguille comprenant la cassette à particules selon la revendication 19.

21. Procédé d'assemblage d'une cassette à particules pour un dispositif à seringue sans aiguille selon l'une des revendications 18 et 19, ledit procédé comprenant :
(a) le scellement d'une première membrane (22) pouvant être rompue à une première partie (20) de cassette ;
(b) le scellement d'une seconde membrane (23) pouvant être rompue à une deuxième partie (21) de cassette ;
(c) la fourniture de particules (24) à ladite première partie (20) de cassette ; et
(d) le fait d'attacher lesdites première et seconde parties de cassette l'une à l'autre afin de créer une chambre confinant lesdites particules fournies (24) entre lesdites première et seconde membranes.

22. Procédé selon la revendication 21, dans lequel lesdites étapes de scellement (a) et (b) comprennent un scellement à chaud.

23. Procédé selon la revendication 21 ou 22, dans lequel l'étape d'attache (d) n'implique pas l'application de chaleur.

24. Procédé selon la revendication 21, 22 ou 23, dans lequel l'étape d'attache (d) est effectuée à la même température que celle de l'étape de fourniture (c).

25. Procédé selon l'une quelconque des revendications 21 à 24, dans lequel ladite première partie de cassette (20) est sensiblement annulaire et définit un réceptacle, lesdites particules étant amenées audit réceptacle dans l'étape (c).

26. Procédé selon l'une quelconque des revendications 21 à 25, dans lequel ladite seconde partie (21) de cassette est sensiblement annulaire et l'étape (d) comprend le montage de ladite seconde partie de cassette concentriquement autour ou à l'intérieur de ladite première partie de cassette.

27. Procédé selon l'une quelconque des revendications 21 à 26, dans lequel l'étape (d) comprend l'établissement d'un ajustement serré entre lesdites première et seconde parties de cassette.

28. Procédé selon l'une quelconque des revendications 21 à 27, dans lequel lesdites première et seconde parties de cassette comprennent chacune des détails correspondants (28, 29) qui réalisent un ajustement par encliquetage lorsque lesdites première et seconde parties de cassette sont attachées et l'étape (d) comprend l'association desdits détails correspondants pour encliqueter ladite seconde partie de cassette sur ladite première partie de cassette.

29. Procédé selon la revendication 28, dans lequel lesdits détails correspondants comprennent un bossage (28) et un évidement (29) et l'étape (d) comprend la mise en place dudit bossage dans ledit évidement.

30. Procédé selon l'une quelconque des revendications 21 à 29, dans lequel les étapes de scellement (a) et (b) comprennent le scellement desdites première et seconde membranes le long de leur périphérie à respectivement des première et seconde parties de cassette sensiblement annulaires.

31. Procédé selon l'une quelconque des revendications 21 à 30, dans lequel l'étape (c) est exécutée avant l'étape (b).

32. Procédé selon l'une quelconque des revendications 21 à 31, dans lequel lesdites première et seconde parties de cassette présentent chacune une face d'appui (27) sensiblement perpendiculaire à la direction dans laquelle les parties sont déplacées pour être attachées et l'étape (d) comprend en outre la mise en contact desdites faces d'appui respectives.

33. Procédé selon l'une quelconque des revendications 21 à 32, dans lequel lesdites première et seconde parties de cassette comprennent chacune une face oblique (26) formant un angle aigu avec la direction dans laquelle les parties sont déplacées pour être attachées et l'étape (d) comprend en outre la mise en contact desdites faces obliques respectives.

34. Procédé selon l'une quelconque des revendications 21 à 33, dans lequel ladite étape d'attache (d) comprend l'introduction d'une troisième partie de cassette (50) afin d'attacher entre elles lesdites première et deuxième parties de cassette.

35. Procédé selon la revendication 34, comprenant en outre le scellement d'une troisième membrane (61) pouvant être rompue à ladite troisième partie de cassette (50) avant l'utilisation de ladite troisième partie de cassette.

36. Procédé selon l'une quelconque des revendications 21 à 35, dans lequel l'étape (d) comprend la rotation des première et deuxième parties de cassette l'une par rapport à l'autre afin de les visser l'une à l'autre.

37. Procédé selon la revendication 36, dans lequel ladite rotation relative est inférieure à 180°.
